# EUROPÄISCHE PATENTSCHRIFT

(11) **EP 3 421 075 B1**
(45) Veröffentlichungstag und Bekanntmachung des Hinweises auf die Patenterteilung: **24.02.2021**
(21) Anmeldenummer: 18176594.2
(22) Anmeldetag: 07.06.2018
(51) Int. Cl.: A61M 39/10, A61M 39/24, A61M 39/26

(54) **MEDIZINISCHE FLUIDVERBINDUNGSVORRICHTUNG**
MEDICAL FLUID CONNECTION DEVICE
DISPOSITIF DE LIAISON FLUIDIQUE MÉDICAL

(30) Priorität: 27.06.2017 DE 102017210795
(43) Veröffentlichungstag der Anmeldung: 02.01.2019
(73) Patentinhaber: B. Braun Melsungen AG, 34212 Melsungen (DE)
(72) Erfinder: ZINDEL, Steffen, 37287 Wehretal (DE); LE, Viet Minh Duc, 34212 Melsungen (DE)
(74) Vertreter: Patentanwälte Ruff, Wilhelm, Beier, Dauster & Partner mbB

(56) Entgegenhaltungen:
- WO-A1-2010/028040
- US-A1- 2014 371 724

## Beschreibung

Die Erfindung betrifft eine medizinische Fluidverbindungsvorrichtung gemäß dem Oberbegriff von Anspruch 1.

Eine Fluidverbindungsvorrichtung ist aus der WO 2010/028040 A1 bekannt. Die bekannte medizinische Fluidverbindungsvorrichtung ist zur fluidleitenden Verbindung medizinischer Schlauchabschnitte, beispielsweise bei einer Infusionstherapie, vorgesehen und weist einen Gehäusekörper mit einem Grundkörper und einem gewindetragenden Verschlussteil auf. Der Gehäusekörper weist eine Einlassöffnung auf, die über einen entlang einer Axialrichtung erstreckten Fluidkanal mit zwei stirnendseitig angeordneten Auslassöffnungen fluidleitend verbunden ist. Der Fluidkanal ist rohrförmig ausgebildet, wobei die Auslassöffnungen jeweils in Form einer Radialbohrung durch eine Wandung des Fluidkanals erstreckt sind. Die Wandung des Fluidkanals in einer Umfangsrichtung umgreifend ist ein weichelastischer, längserstreckter Dichtköper vorgesehen. Der Dichtkörper dichtet in einer Dichtposition die Auslassöffnungen fluiddicht ab. In einem mit einer komplementären Anschlusseinheit verbundenen Zustand der Fluidverbindungsvorrichtung ist der Dichtköper entlang der Axialrichtung auf der Wandung des Fluidkanals in eine Stauchposition gestaucht und gibt derart die Auslassöffnungen frei. Der Gehäusekörper weist einen Luer-Außenkonus auf, der zur fluiddichten Aufnahme eines Luer-Innenkonus der komplementären Anschlusseinheit vorgesehen ist. Der Luer-Außenkonus ist durch eine entsprechend konische Ausgestaltung eines stirnendseitigen Bereichs der Wandung des Fluidkanals ausgebildet.

Eine weitere medizinische Fluidverbindungsvorrichtung ist aus der US 2014/0371724 A1 bekannt. Die bekannte Fluidverbindungsvorrichtung ist zur fluidleitenden Verbindung mit einer komplementären Anschlusseinheit vorgesehen und weist einen Gehäusekörper mit einer Einlassöffnung und einer Auslassöffnung auf. Zudem ist ein weichelastischer Dichtkörper vorgesehen, der entlang seiner Axialrichtung stauchbar ausgebildet ist. Zur Verbindung mit der komplementären Anschlusseinheit weist die medizinische Fluidverbindungsvorrichtung einen Luer-Außenkonus auf. Dieser ist an einem stirnendseitigen Abschnitt des Gehäusekörpers ausgebildet.

Aufgabe der Erfindung ist es, eine medizinische Fluidverbindungsvorrichtung der eingangs genannten Art zu schaffen, die gegenüber dem Stand der Technik verbesserte Eigenschaften aufweist.

Diese Aufgabe wird dadurch gelöst, dass der Luer-Außenkonus durch einen Wandabschnitt des weichelastischen Dichtkörpers ausgebildet ist. Durch die erfindungsgemäße Lösung wird zum einen eine funktions- und fertigungsgerechte Ausgestaltung der Fluidverbindungsvorrichtung erreicht. Dies, da auf eine konische Ausgestaltung der Wandung des Fluidkanals zur Ausbildung des Luer-Außenkonus verzichtet werden kann. Derart kann eine besonders leichtgängige Bewegbarkeit des Dichtkörpers zwischen der Dicht- und der Stauchposition erreicht werden, da eine ansonsten üblicherweise hierbei auftretende - durch eine konusförmige Ausgestaltung der Wandung des Fluidkanals bewirkte - Axialkraft- und/oder Umfangskrafteinwirkung auf den Dichtkörper vermieden wird. Zum anderen erlaubt die erfindungsgemäße Lösung eine verbesserte Dichtigkeit der Luer-Verbindung zwischen der Fluidverbindungsvorrichtung und der komplementären Anschlusseinheit. Dies, da der Luer-Außenkonus an dem weichelastischen Dichtkörper ausgebildet ist. Derart können etwaig vorhandene Form- und/oder Lagetoleranzen der Luer-Verbindung zwischen der Fluidverbindungsvorrichtung und der Anschlusseinheit mittels einer elastischen Deformation des Dichtkörpers ausgeglichen werden. Auf diese Weise kann eine Undichtigkeit vermieden werden. Die Wandung des Fluidkanals kann in Form eines längserstreckten Hohlzylinders, einer Röhre, einer Hülse oder dergleichen ausgebildet sein. Vorteilhafterweise kann eine Außenwandfläche der Wandung des Fluidkanals in Form einer im Wesentlichen geraden Kreiszylinderfläche ausgebildet sein. Der weichelastische Dichtkörper kann eine hülsen- oder röhrenartige Grundform aufweisen. Vorteilhafterweise kann eine Innenwandfläche des Dichtkörpers wenigstens abschnittsweise an der Außenwandfläche des Fluidkanals in Radialrichtung festgelegt sein. Insoweit kann der Dichtkörper bei einer Verlagerung zwischen der Dicht- und der Stauchposition auf der Wandung des Fluidkanals in Radialrichtung abgestützt und in Axialrichtung gleitend geführt sein. Der Dichtkörpers kann eine Werkstoffzusammensetzung aufweisen, die ein Elastomer, insbesondere ein Silikonkautschuk, beinhaltet. Von Vorteil ist der Dichtkörper derart ausgestaltet, dass dieser nach einer Deformation von der Dicht- in die Stauchposition - und nach Entfernen der komplementären Anschlusseinheit - elastisch in die Dichtposition zurückfedert, wobei die Auslassöffnung selbsttätig abgedichtet und somit ein Fluidpfad durch die Fluidverbindungsvorrichtung selbsttätig verschlossen wird. Die in dieser Beschreibung verwendeten Richtungsangaben der Axial-, der Radial- und der Umfangsrichtung beziehen sich auf ein Koordinatensystem, dessen Axialkomponente koaxial oder parallel mit einer Längsachse der Fluidverbindungsvorrichtung orientiert ist.

In Ausgestaltung der Erfindung weist der Dichtkörper wenigstens eine Durchlassöffnung auf, die in der Stauchposition derart zumindest abschnittsweise überdeckend zu der wenigstens einen Auslassöffnung angeordnet ist, dass diese wenigstens abschnittsweise freigegeben und eine fluidleitende Verbindung zwischen der Einlassöffnung und der Durchlassöffnung bewirkt ist. Demzufolge ist in der Stauchposition des Dichtkörpers ein durch die Fluidverbindungsvorrichtung erstreckter Fluidpfad freigegeben, so dass ein entsprechendes Fluid, beispielsweise eine Infusionslösung, zwischen der Einlassöffnung, der Auslassöffnung und der Durchlassöffnung strömen kann. Vorteilhafterweise kann die wenigstens eine Durchlassöffnung an den Luer-Außenkonus angrenzend angeordnet ist. Diese Ausgestaltung der Erfindung erlaubt eine besonders funktionssichere Abdichtung des Fluidpfads durch die Fluidverbindungsvorrichtung.

In weiterer Ausgestaltung der Erfindung ist die wenigstens eine Durchlassöffnung in Gestalt eines Durchlassschlitzes ausgebildet und/oder die wenigstens eine Auslassöffnung ist in Gestalt eines Auslassschlitzes ausgebildet. Von Vorteil kann der Durchlassschlitz und/oder der Auslassschlitz im Wesentlichen entlang der Axialrichtung erstreckt sein. Der Durchlassschlitz kann entlang seiner Umfangsrichtung durch entsprechende Wandabschnitte des Dichtkörpers vollständig umlaufend berandet sein oder an wenigstens einer seiner Stirnendseiten offen sein. Vorteilhafterweise können wenigstens zwei Durchlassschlitze entlang der Umfangsrichtung des Dichtkörpers zueinander versetzt angeordnet sein. Dementsprechend können von Vorteil wenigstens zwei Auslassschlitze vorgesehen sein.

Weiter gemäß der Erfindung ist die wenigstens eine Auslassöffnung im Wesentlichen radial durch die Wandung des Fluidkanals erstreckt. Demzufolge ist die Auslassöffnung in Form einer Radialöffnung ausgebildet. Sofern die Auslassöffnung in Gestalt eines Auslassschlitzes ausgebildet ist, ist es vorteilhaft, wenn der Auslassschlitz über wenigstens 25 % der Axiallänge der Wandung des Fluidkanals erstreckt ist.

In weiterer Ausgestaltung der Erfindung weist der Dichtkörper einen Verschlussabschnitt auf, der eine stirnendseitige Axialöffnung des Fluidkanals fluiddicht abdichtet. Die derartige fluiddichte Abdichtung der Axialöffnung des Fluidkanals ist wenigstens in der Dichtposition des Dichtkörpers bewirkt. Vorteilhafterweise kann der Verschlussabschnitt derart ausgestaltet sein, dass die Axialöffnung zudem in der Stauchposition - und insoweit in einem mit der komplementären Anschlusseinheit verbundenen Zustand - fluiddicht abgedichtet ist. Der Verschlussabschnitt kann die Axialöffnung stirnendseitig abdeckend angeordnet sein und insoweit einen wirksamen Querschnitt aufweisen, der größer ist als der Querschnitt der Axialöffnung des Fluidkanals. Alternativ kann der Verschlussabschnitt einen Außendurchmesser aufweisen, der im Wesentlichen einem Innendurchmesser der Axialöffnung des Fluidkanals entspricht, wobei die fluiddichte Abdichtung durch eine Anlage des Verschlussabschnitts in radialer Richtung an einer Innenwandung des Fluidkanals bewirkt ist.

In weiterer Ausgestaltung der Erfindung ist der Verschlussabschnitt derart ausgebildet, dass dieser bei einer Verlagerung des Dichtkörpers zwischen der Dicht- und der Stauchposition entlang des Fluidkanals in dem Fluidkanal beweglich ist. Der Verschlussabschnitt kann in Gestalt eines Stopfens, eines Pfropfens, eines Zapfens oder dergleichen ausgebildet sein. Bei einer Verlagerung des Dichtkörpers zwischen der Dichtposition und der Stauchposition kann der Verschlussabschnitt in Axialrichtung gleitbeweglich und in Radialrichtung fluiddicht an einer Innenwandung des Fluidkanals abgestützt sein. Diese Ausgestaltung der Erfindung gewährleistet eine besonders funktionssichere Abdichtung der Axialöffnung des Fluidkanals.

In weiterer Ausgestaltung der Erfindung weist der Dichtkörper einen Profilabschnitt auf, der derart formschlüssig mit einem komplementären Gegenprofilabschnitt des Gehäusekörpers verbunden ist, dass der Dichtkörper in Umfangsrichtung an dem Gehäusekörper, insbesondere an der Wandung des Fluidkanals, festgelegt ist. Es ist vorteilhaft, wenn der Gegenprofilabschnitt durch eine entsprechende Profilierung der Wandung des Fluidkanals ausgebildet ist. Der Gegenprofilabschnitt kann in Gestalt einer Führungsnut, der Profilabschnitt dementsprechend in Gestalt eines komplementären Führungselements ausgebildet sein oder umgekehrt. Infolge der formschlüssigen Verbindung in Umfangsrichtung zwischen dem Profilabschnitt und dem Gegenprofilabschnitt wird einem ungewollten Verdrehen des Dichtkörpers bei einer Verlagerung zwischen der Dicht- und der Stauchposition entgegengewirkt. Diese Ausgestaltung der Erfindung erlaubt demzufolge eine besonders toleranzgerechte Führung des Dichtkörpers gegenüber der Wandung des Fluidkanals. Auf diese Weise kann ein besonders zuverlässiges Verschließen und Öffnen des durch die Fluidverbindungsvorrichtung erstreckten Fluidpfads erreicht werden.

In weiterer Ausgestaltung der Erfindung weist der Profilabschnitt wenigstens ein Gleitelement auf, das in einen Führungsschlitz des Gegenprofilabschnitts eingreift. Das Gleitelement kann in Form einer Gleitleiste, eines Gleitschuhs, eines Gleitsteins oder dergleichen ausgebildet sein. Der Führungsschlitz kann vorteilhafterweise im Wesentlichen entlang der Axialrichtung erstreckt sein. Es ist zudem vorteilhaft, wenn der Führungsschlitz in die Wandung des Fluidkanals eingebracht ist. Eine besonders toleranzgerechte Führung des Dichtkörpers gegenüber der Wandung des Fluidkanals kann erreicht werden, wenn wenigstens zwei in Umfangsrichtung zueinander versetzt angeordnete Gleitelemente und dementsprechend wenigstens zwei in Umfangsrichtung zueinander versetzt angeordnete Führungsschlitze vorgesehen sind.

In weiterer Ausgestaltung der Erfindung weist der Fluidkanal einen Aufnahmeabschnitt auf, der zur Aufnahme eines längserstreckten Aufnahmefortsatzes der komplementären Anschlusseinheit vorgesehen ist. Der Aufnahmeabschnitt kann einen Wandabschnitt der Innenwandung des Fluidkanals umfassen. Der Aufnahmefortsatz kann in Form eines Dorns, eines Stifts, einer Röhre oder dergleichen ausgebildet sein. Vorteilhafterweise kann der Aufnahmeabschnitt einen stirnendseitigen Abschnitt des Fluidkanals umfassen. Diese Ausgestaltung der Erfindung erlaubt eine besonders toleranzgerechte Ausrichtung der Fluidverbindungsvorrichtung und der komplementären Anschlusseinheit zueinander, so dass dementsprechend eine besonders toleranzgerechte Ausrichtung des Luer-Außenkonus gegenüber dem Luer-Innenkonus erreicht wird. Auf diese Weise können ungewollte Undichtigkeiten vermieden werden.

In weiterer Ausgestaltung der Erfindung weist der Gehäusekörper einen Gewindeabschnitt, insbesondere in Gestalt eines Innengewindes, auf, der zur Verbindung mit einem Gegengewindeabschnitt, insbesondere in Form eines Außengewindes, der komplementären Anschlusseinheit vorgesehen ist. Der Gewindeabschnitt kann in Gestalt einer Gewindebohrung, die gegenüber dem Gehäusekörper unbeweglich ist, oder in Gestalt einer gegenüber dem Gehäusekörper beweglichen Überwurfmutter, einer Gewindehülse oder dergleichen ausgebildet sein. Durch diese Ausgestaltung der Erfindung wird einem unbeabsichtigten Lösen der Verbindung zwischen dem Luer-Außenkonus und dem Luer-Innenkonus entgegengewirkt.

Die der Erfindung zugrunde liegende Aufgabe wird auch dadurch gelöst, dass die Fluidverbindungsvorrichtung aus dem Dichtkörper und dem Gehäusekörper besteht. Insoweit kann die Fluidverbindungsvorrichtung aus lediglich zwei Komponenten, nämlich dem Dichtkörper und dem Gehäusekörper, bestehen. Durch die derartige Reduktion der Anzahl der Bauteile kann eine besonders einfache und kostengünstige Fertigung erreicht werden.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer Perspektivdarstellung eine Ausführungsform einer erfindungsgemäßen Fluidverbindungsvorrichtung, die insbesondere zur fluiddichten Verbindung von Schlauchabschnitten bei einer Infusionstherapie vorgesehen ist,
- Fig. 2a: in einer schematischen Perspektivdarstellung entsprechend Fig. 1 einen weichelastischen Dichtkörper der Fluidverbindungsvorrichtung nach Fig. 1 in einem elastisch undeformierten Zustand (Dichtposition),
- Fig. 2b und 2c: den Dichtkörper nach den Fig. 1 und 2a in einer schematischen Längsschnittdarstellung in einer Schnittebene Y-Z gemäß Fig. 1 in dem elastisch undeformiertem Zustand (Fig. 2b) und in einem elastisch gestauchten Zustand (Stauchposition) (Fig. 2c),
- Fig. 3a und 3b: in einer schematischen Längsschnittdarstellung in einer Schnittebene Y-Z gemäß Fig. 1 die Fluidverbindungsvorrichtung nach Fig. 1 in einem fluiddicht verschlossenen Zustand (Fig. 3a) und in einem geöffneten Zustand (Fig. 3b), wobei der Dichtkörper die Stauchposition einnimmt,
- Fig. 4a und 4b: in einer weiteren schematischen Längsschnittdarstellung in einer Schnittebene X-Y gemäß Fig. 1 die Fluidverbindungsvorrichtung nach den Fig. 1 und 3a sowie 3b in fluiddicht verschlossenem Zustand (Fig. 4a) und dem entsprechend Fig. 3b geöffneten Zustand (Fig. 4b) und
- Fig. 5: in einer schematischen Längsschnittdarstellung in einer Schnittebene X-Y gemäß Fig. 1 die Fluidverbindungsvorrichtung nach den Fig. 1, 3a, 3b, 4a sowie 4b in einem mit einer komplementären Anschlusseinheit verbundenen Zustand.

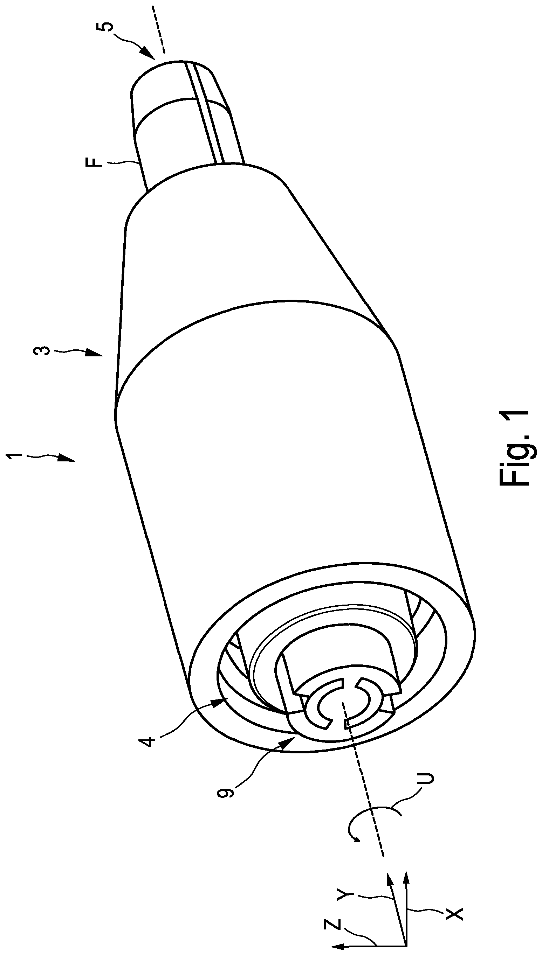

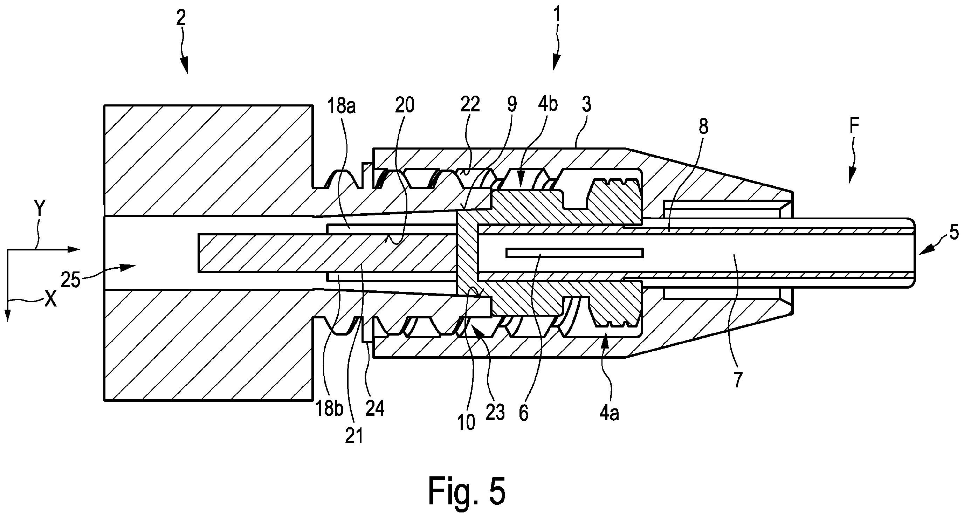

Eine medizinische Fluidverbindungsvorrichtung 1 nach den Fig. 1, 3a bis 5 ist in Gestalt eines männlichen Luer-Anschlusskonnektors ausgebildet und zur fluidleitenden Verbindung zweier medizinischer Schlauchleitungsabschnitte vorgesehen. Zu diesem Zweck ist die medizinische Fluidverbindungsvorrichtung 1 auf noch näher zu erläuternde Art und Weise fluiddicht mit einer komplementären Anschlusseinheit 2 (Fig. 5), die in Gestalt eines weiblichen Luer-Anschlusskonnektors ausgebildet ist, verbindbar.

Die Fluidverbindungsvorrichtung 1 besteht aus lediglich zwei Bauteilen, nämlich einem Gehäusekörper 3 und einem weichelastischen Dichtkörper 4.

Der Gehäusekörper 3 weist eine stirnendseitig angeordnete Einlassöffnung 5 und wenigstens eine Auslassöffnung 6 auf. Die Einlassöffnung 5 und die Auslassöffnung 6 sind über einen im Wesentlichen entlang einer Axialrichtung Y erstreckten Fluidkanal 7 fluidleitend miteinander verbunden. Die Einlassöffnung 5 ist an einem stirnendseitigen Gehäusefortsatz F des Gehäusekörpers 3 angeordnet, der zur fluiddichten Verbindung mit einem nicht näher bezeichneten medizinischen Schlauchabschnitt vorgesehen ist. Der weichelastische Dichtkörper 4 ist in seiner Grundform in Gestalt einer Hülse ausgebildet, die eine Wandung 8 des Fluidkanals 7 in einer Umfangsrichtung U wenigstens abschnittsweise umgreift und entlang der Axialrichtung Y gegenüber der Wandung 8 beweglich geführt ist. Mit anderen Worten: Der Dichtkörper 4 ist entlang der Axialrichtung Y auf die Wandung 8 des Fluidkanals 7 aufgesteckt und insoweit in einer radialen Richtung an der Wandung 8 abgestützt.

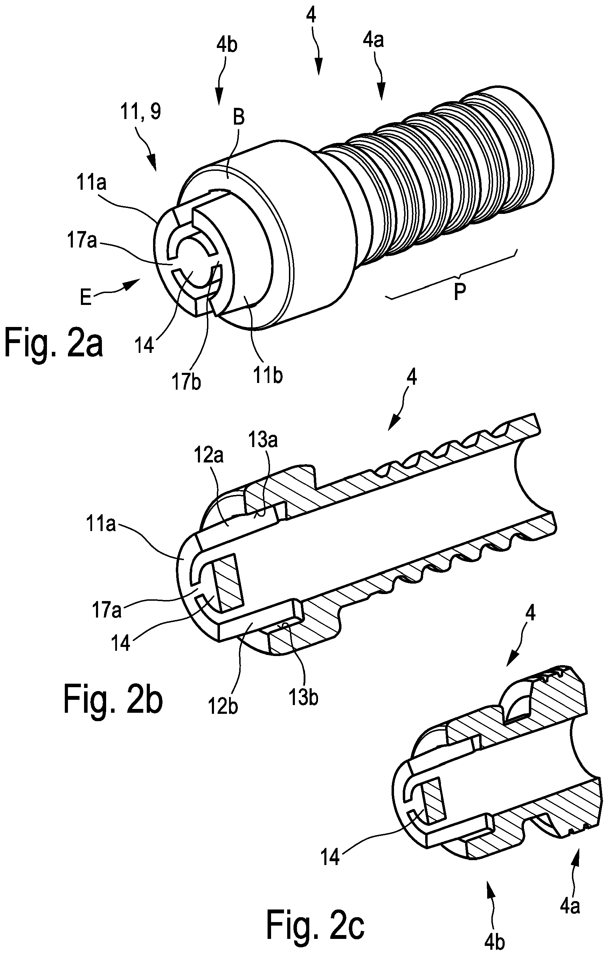

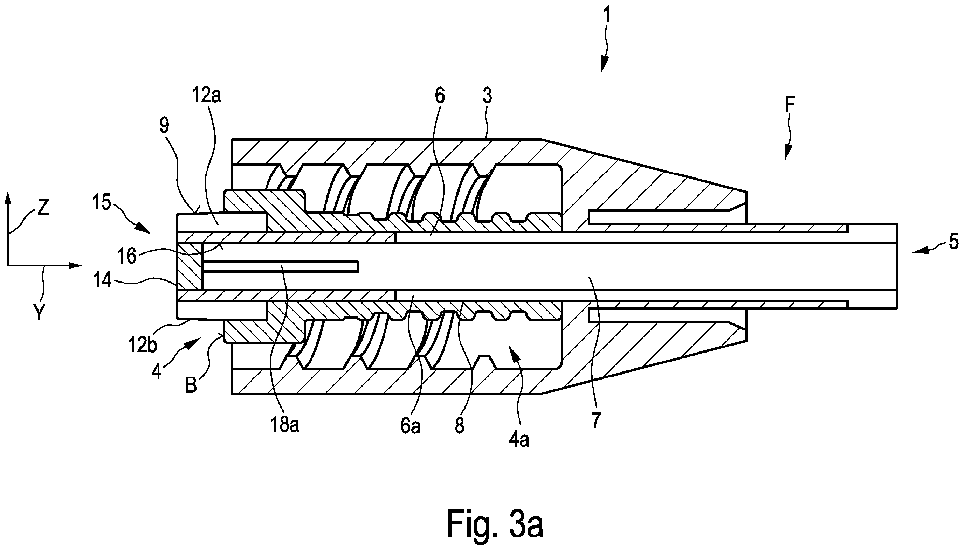

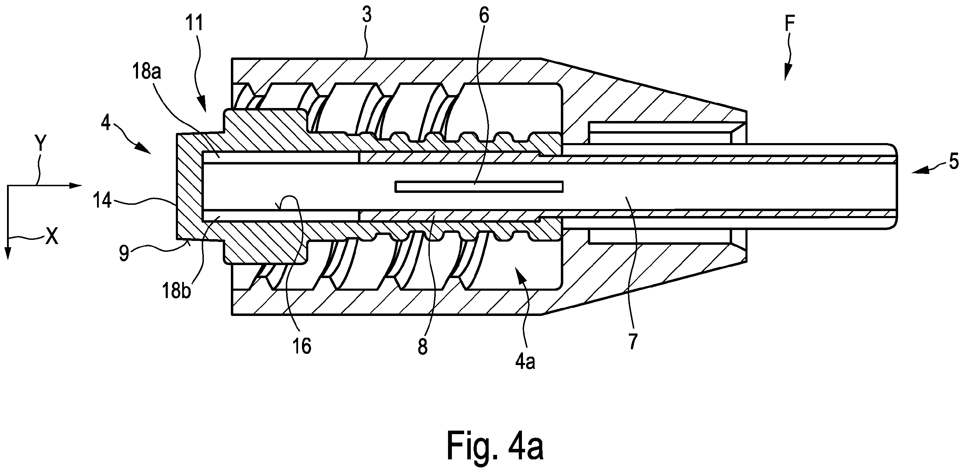

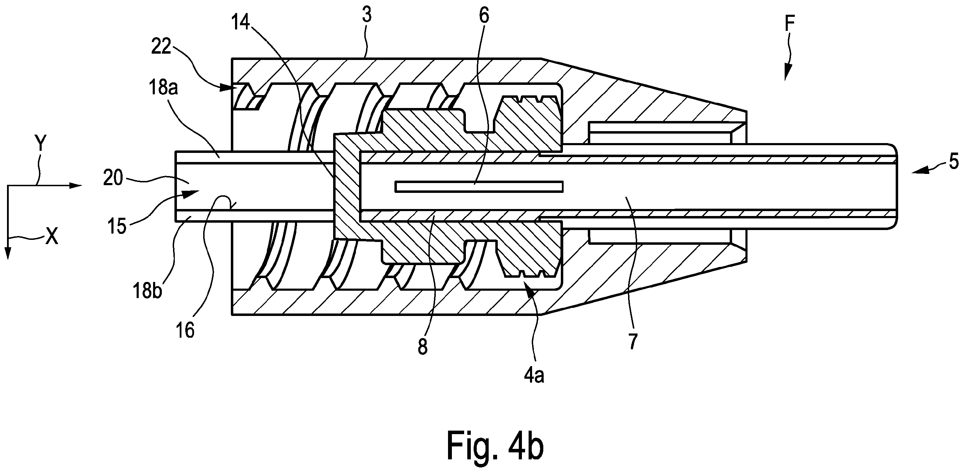

Wie insbesondere anhand der Fig. 3a und 4a ersichtlich ist, nimmt der Dichtkörper 4 in einem nicht verbundenen Zustand der Fluidverbindungsvorrichtung 1 eine Dichtposition ein. In dieser Dichtposition dichtet der Dichtkörper 4 die wenigstens eine Auslassöffnung 6 fluiddicht ab, so dass ein zwischen der Einlassöffnung 5 und der Auslassöffnung 6 erstreckter Fluidpfad fluiddicht abgedichtet ist. Demgegenüber nimmt der Dichtkörper 4 in einem mit der komplementären Anschlusseinheit 2 verbundenen Zustand der Fluidverbindungsvorrichtung 1 eine Stauchposition ein (Fig. 2c, 3b, 4b sowie 5). In dieser Stauchposition ist der Dichtkörper 4 entlang der Axialrichtung Y derart elastisch gestaucht, dass die wenigstens eine Auslassöffnung 6 und somit der durch die Fluidverbindungsvorrichtung 1 erstreckte Fluidpfad freigegeben ist.

Zur fluiddichten Verbindung der Fluidverbindungsvorrichtung 1 mit der Anschlusseinheit 2 ist ein als solcher bekannter Luer-Außenkonus 9 vorgesehen. Dieser Luer-Außenkonus ist zur Aufnahme eines Luer-Innenkonus 10 der Anschlusseinheit 2 vorgesehen. Der Luer-Außenkonus 9 und der Luer-Innenkonus 10 bilden insoweit ein im Bereich der Medizintechnik als solches bekanntes Luer-Verbindungssystem aus.

Erfindungsgemäß ist der Luer-Außenkonus 9 durch einen Wandabschnitt 11 des weichelastischen Dichtkörpers 4 ausgebildet. Wie insbesondere anhand der Fig. 2a bis 2c ersichtlich ist, ist der Wandabschnitt 11 stirnendseitig an dem der Einlassöffnung 5 abgewandten Ende des Dichtkörpers 4 angeordnet. Der Wandabschnitt 11 ist ein in Umfangsrichtung U erstreckter Wandabschnitt des Dichtkörpers. In Axialrichtung Y ist der Wandabschnitt durch einen radial erstreckten Bund B begrenzt. Zudem umfasst der Wandabschnitt 11 zwei in Umfangsrichtung U voneinander getrennte Teilwandabschnitte 11a und 11b, wobei die Trennung zwischen diesen beiden Teilwandabschnitten auf noch näher zu erläuternde Art und Weise ausgebildet ist. Der Luer-Außenkonus 9 erstreckt sich in Axialrichtung Y über in etwa 1/9 der Gesamtlänge des Dichtkörpers 4 bezogen auf die undeformierte Dichtposition des Dichtkörpers 4 (Fig. 2a).

Wie weiter insbesondere anhand der Fig. 2a bis 2c ersichtlich ist, weist der Dichtkörper 4 einen Stauchabschnitt 4a sowie einen Kopplungsabschnitt 4b auf. Der Stauchabschnitt 4a ist im Vergleich zu dem Kopplungsabschnitt 4b dünnwandig ausgestaltet und weist infolge einer Profilierung P eine ziehharmonikaartige Gestalt auf. In Verbindung mit der weichelastischen Materialzusammensetzung des Dichtkörpers 4, die insbesondere Silikonkautschuk aufweisen kann, erlaubt die derartige Ausgestaltung des Stauchabschnitts 4a eine funktionsgerechte elastische Stauchbarkeit des Dichtkörpers 4 entlang der Axialrichtung Y. Demgegenüber ist der Kopplungsabschnitt 4b vergleichsweise dickwandig ausgeführt und umfasst insbesondere den Luer-Außenkonus 9 sowie dem Bund B. In der Stauchposition des Dichtkörpers 4 ist der Kopplungsabschnitt 4b im Wesentlichen undeformiert, wohingegen der Stauchabschnitt 4a auf in etwa 1/3 seiner undeformierten Ausgangslänge zusammengestaucht ist.

Weiter weist der Dichtkörper 4 zwei Durchlassöffnungen 12a, 12b auf. Es ist selbstverständlich auch möglich, dass lediglich eine Durchlassöffnung an dem Dichtkörper 4 vorgesehen ist. In der Stauchposition des Dichtkörpers 4 ist die Durchlassöffnung derart zumindest abschnittsweise überdeckend zu der wenigstens einen Auslassöffnung 6 angeordnet, dass diese wenigstens abschnittsweise freigegeben und somit eine fluidleitende Verbindung zwischen der Einlassöffnung 5 und den beiden Durchlassöffnungen bewirkt ist. Die Durchlassöffnungen 12a, 12b sind jeweils in Gestalt eines Durchlassschlitzes ausgebildet. Die Durchlassschlitze 12a, 12b sind ausgehend von einer axialen Stirnendfläche E entlang der Axialrichtung Y in den Wandabschnitt 11 eingebracht. Insoweit unterteilen die Durchlassöffnungen 12a, 12b den Wandabschnitt 11 in die beiden Teilwandabschnitte 11a und 11b. Im Bereich des Luer-Außenkonus 9 bzw. des Wandabschnitts 11 sind die Durchlassöffnungen 12a, 12b in radialer Richtung offen. In einem sich in der Axialrichtung Y an den Luer-Außenkonus 9 anschließenden Bereich sind die Durchlassöffnungen 12a, 12b in radialer Richtung jeweils durch einen Wandabschnitt 13a, 13b des Dichtkörpers 4, insbesondere des Kopplungsabschnitts 4b, abgedeckt.

Entsprechend zu der schlitzförmigen Ausgestaltung der Durchlassöffnung 12a ist die wenigstens eine Auslassöffnung 6 in Gestalt eines Auslassschlitzes ausgebildet. Zudem ist eine zweite Auslassöffnung 6a vorgesehen, die der Durchlassöffnung 12b zugeordnet und ebenfalls in Gestalt eines Auslassschlitzes ausgebildet ist. Die beiden Auslassschlitze 6, 6a sind - ebenso wie die Durchlassschlitze 12a, 12b - in Umfangsrichtung U in etwa um 180° zueinander versetzt angeordnet. Die Auslassöffnungen 6, 6a sind radial durch die Wandung 8 des Fluidkanals 7 erstreckt und insoweit in Gestalt von jeweils einer Radialöffnung ausgebildet. Die Länge der Auslassöffnungen 6, 6a in Bezug auf die Axialrichtung Y entspricht in etwa 50 % der undeformierten Länge des Dichtkörpers 4.

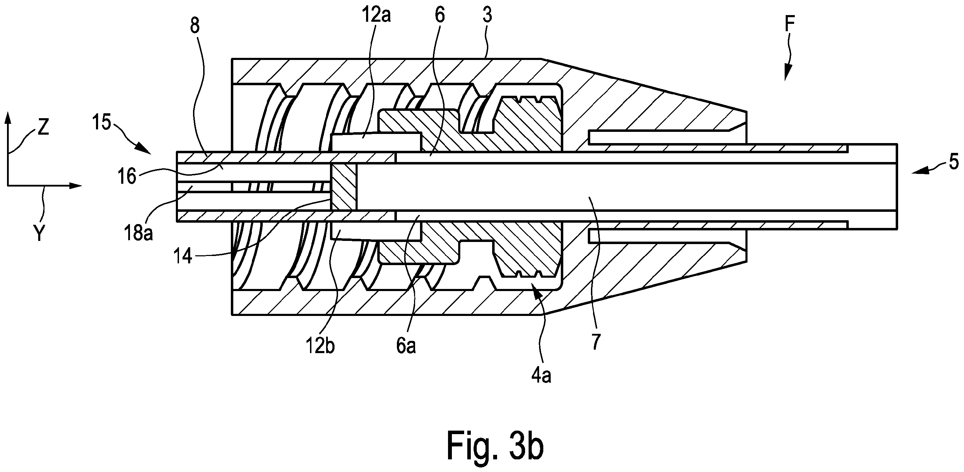

Weiter weist der Dichtkörper 4 einen Verschlussabschnitt 14 auf. Der Verschlussabschnitt 14 ist in Gestalt eines kreiszylinderförmigen Stopfens ausgebildet und zur Abdichtung einer stirnendseitigen Axialöffnung 15 des Fluidkanals 7 vorgesehen. Wie insbesondere anhand der Fig. 3a und 3b ersichtlich ist, ist der Verschlussabschnitt 14 derart ausgebildet, dass dieser bei einer Verlagerung des Dichtkörpers 4 zwischen der Dicht- und der Stauchposition entlang des Fluidkanals 7 in dem Fluidkanal 7 beweglich ist. Bei einer derartigen Verlagerung des Dichtkörpers 4 liegt der Verschlussabschnitt 14 in radialer Richtung an einer Innenwandung 16 des Fluidkanals 7 an, so dass die Axialöffnung 15 permanent abgedichtet ist.

Weiter weist der Dichtkörper 4 zwei Profilabschnitte 17a, 17b auf. Es ist selbstverständlich auch möglich, dass lediglich ein Profilabschnitt vorgesehen ist. Die beiden Profilabschnitte 17a, 17b sind formschlüssig mit jeweils einem komplementären Gegenprofilabschnitt 18a, 18b des Gehäusekörpers 3 verbunden. Auf diese Weise ist der Dichtkörper 4 in Umfangsrichtung U an dem Gehäusekörper 3 festgelegt. Die Profilabschnitte 17a, 17b sind jeweils in Gestalt eines Gleitelements ausgebildet. Dementsprechend sind die Gegenprofilabschnitte 18a, 18b jeweils in Gestalt eines in Axialrichtung Y erstreckten Führungsschlitzes ausgebildet. Die Führungsschlitze 18a, 18b sind jeweils ausgehend von dem der Einlassöffnung 5 abgewandten Stirnende des Fluidkanals 7 in dessen Wandung 8 eingebracht. Die Führungsschlitze 18a, 18b erstrecken sich in Bezug auf die Axialrichtung Y jeweils über eine Länge, die in etwa 50 % der undeformierten Länge des Dichtkörpers 4 entspricht. Im Übrigen bilden die Profilabschnitte 17a, 17b jeweils eine Materialanbindung zwischen dem Verschlussabschnitt 14 und dem Luer-Außenkonus 9 bzw. dem Wandabschnitt 11. Wie weiter insbesondere anhand Fig. 2a ersichtlich ist, sind die Profilabschnitte 17a, 17b in Umfangsrichtung U um 180° versetzt zueinander angeordnet. Hierbei beträgt der entsprechende Winkelversatz zwischen den Profilabschnitten 17a, 17b und den Durchlassöffnungen 12a, 12b jeweils in etwa 90°.

Wie weiter insbesondere anhand Fig. 5 ersichtlich ist, weist der Fluidkanal 7 einen Aufnahmeabschnitt 20 auf. Der Aufnahmeabschnitt 20 ist zur Aufnahme eines längserstreckten Aufnahmefortsatzes 21 der komplementären Anschlusseinheit 2 vorgesehen. Der Aufnahmefortsatz 21 ist in Gestalt eines kreiszylindrischen Stiftes ausgestaltet. Der Außendurchmesser des Aufnahmefortsatzes 21 entspricht im Wesentlichen einem Innendurchmesser des Aufnahmeabschnitts 20.

Zudem weist der Gehäusekörper 3 einen Gewindeabschnitt 22 auf. Der Gewindeabschnitt 22 ist in Gestalt eines Innengewindes ausgestaltet. Das Innengewinde 22 ist zur Verbindung mit einem Gegengewindeabschnitt 23 in Gestalt eines Außengewindes vorgesehen, das stirnendseitig den Aufnahmefortsatz 21 umgreifend an der Anschlusseinheit 2 angeordnet ist.

Zur fluidleitenden Verbindung der medizinischen Fluidverbindungsvorrichtung 1 mit der komplementären Anschlusseinheit 2 wird zunächst der Luer-Innenkonus 10 der Anschlusseinheit 2 an dem Luer-Außenkonus 9 der Fluidverbindungsvorrichtung zur Anlage gebracht. Hierbei wird der Luer-Innenkonus 10 in Axialrichtung Y auf den Wandabschnitt 11 des Dichtkörpers 4 aufgesteckt, bis eine Anlage an dem Bund B des Kopplungsabschnitts 4b hergestellt ist. Weiter wird der Gegengewindeabschnitt 23 mit dem Gewindeabschnitt 22 verschraubt, wobei der Dichtkörper 4 in axialer Richtung ausgehend von der Dichtposition (Fig. 3a, 4a) entlang der Axialrichtung Y in die Stauchposition gestaucht wird. Hierbei tritt zudem der Aufnahmefortsatz 21 in den Aufnahmeabschnitt 20 des Fluidkanals 7 ein und der Verschlussabschnitt 14 bewegt sich ausgehend von der stirnendseitigen Axialöffnung 15 des Fluidkanals entlang der Innenwandung 16 in dem Fluidkanal 7. Hierbei wird der Dichtkörper 4 durch das Eingreifen der Gleitelemente 17a, 17b in die Führungsschlitze 18a bzw. 18b in Umfangsrichtung gegenüber dem Gehäusekörper 3 festgelegt. In einer durch einen Anlaufbund 24 des Gegengewindeabschnitts 23 festgelegten Position ist eine funktionsgemäße Verbindung zwischen der Fluidverbindungsvorrichtung 1 und der Anschlusseinheit 2 hergestellt. In dieser sind die Auslassschlitze 6, 6a durch eine entsprechende Positionierung der Durchlassschlitze 12a bzw. 12b wenigstens abschnittsweise freigegeben. Auf diese Weise ist eine fluidleitende Verbindung zwischen der Einlassöffnung 5, den Auslassschlitzen 6, 6a, den Durchlassöffnungen 12, 12b sowie einem Ableitungskanal 25 der Anschlusseinheit 2 hergestellt. An dem der Fluidverbindungsvorrichtung 1 abgewandten Stirnendbereich des Ableitungskanals 25 kann ein nicht näher ersichtlicher Schlauchabschnitt angeordnet sein, der wiederum fluidleitend mit beispielsweise einem Infusionsbehälter oder dergleichen verbunden sein kann. Auf diese Weise kann ein medizinisches Fluid ausgehend von der Einlassöffnung 5 zu dem Ableitungskanal 25 gelangen oder umgekehrt.

Zur Trennung der Fluidverbindungsvorrichtung 1 von der Anschlusseinheit 2 wird die Schraubverbindung zwischen dem Gewindeabschnitt 22 und dem Gegengewindeabschnitt 23 manuell gelöst. Infolge der weichelastischen Ausgestaltung des Dichtkörpers 4 bewirkt dieser eine entlang der Axialrichtung Y gerichtete Rückstellkraft. Insoweit nimmt der Dichtkörper 4 beim Entfernen der komplementären Anschlusseinheit 2 ausgehend von der elastisch gestauchten Dichtposition selbsttätig die elastisch undeformierte Dichtposition ein. Auf diese Weise wird der Fluidpfad zwischen der Einlassöffnung 5 und dem Ableitungskanal 25 selbsttätig verschlossen.

## Patentansprüche

1. Medizinische Fluidverbindungsvorrichtung (1) zur fluidleitenden Verbindung mit einer komplementären Anschlusseinheit (2), mit einem Gehäusekörper (3) mit einer Einlassöffnung (5) und wenigstens einer Auslassöffnung (6, 6a), die über einen im Wesentlichen entlang einer Axialrichtung (Y) erstreckten Fluidkanal (7) fluidleitend miteinander verbunden sind, und einem weichelastischen Dichtkörper (4), der eine Wandung (8) des Fluidkanals (7) in einer Umfangsrichtung (U) wenigstens abschnittsweise umgreift und entlang der Axialrichtung (Y) gegenüber der Wandung (8) des Fluidkanals (7) beweglich geführt ist, wobei der Dichtkörper (4) in einem nicht verbundenen Zustand der Fluidverbindungsvorrichtung (1) eine Dichtposition einnimmt und die wenigstens eine Auslassöffnung (6, 6a), die im Wesentlichen radial durch die Wandung (8) des Fluidkanals (7) erstreckt ist, fluiddicht abdichtet und in einem mit der komplementären Anschlusseinheit (2) verbundenen Zustand der Fluidverbindungsvorrichtung (1) im Bereich eines Stauchabschnitts (4a) entlang der Axialrichtung (Y) derart elastisch in eine Stauchposition gestaucht ist, dass die wenigstens eine Auslassöffnung (6, 6a) freigegeben ist, wobei ein Luer-Außenkonus (9) zur fluiddichten Verbindung mit einem Luer-Innenkonus (10) der komplementären Anschlusseinheit (2) vorgesehen ist, **dadurch gekennzeichnet, dass** der Luer-Außenkonus (9) durch einen Wandabschnitt (11) des weichelastischen Dichtkörpers (4) ausgebildet ist.

2. Medizinische Fluidverbindungsvorrichtung (1) nach Anspruch 1, **dadurch gekennzeichnet, dass** der Dichtkörper (4) wenigstens eine Durchlassöffnung (12a, 12b) aufweist, die in der Stauchposition derart zumindest abschnittsweise überdeckend zu der wenigstens einen Auslassöffnung (6, 6a) angeordnet ist, dass diese wenigstens abschnittsweise freigegeben und eine fluidleitende Verbindung zwischen der Einlassöffnung (5) und der Durchlassöffnung (12a, 12b) bewirkt ist.

3. Medizinische Fluidverbindungsvorrichtung (1) nach Anspruch 2, **dadurch gekennzeichnet, dass** die wenigstens eine Durchlassöffnung (12a, 12b) in Gestalt eines Durchlassschlitzes ausgebildet ist und/oder die wenigstens eine Auslassöffnung (6, 6a) in Gestalt eines Auslassschlitzes ausgebildet ist.

4. Medizinische Fluidverbindungsvorrichtung (1) nach einem der vorhergehenden Ansprüche, **dadurch gekennzeichnet, dass** der Dichtkörper (4) einen Verschlussabschnitt (14) aufweist, der eine stirnendseitige Axialöffnung (15) des Fluidkanals (7) fluiddicht abdichtet.

5. Medizinische Fluidverbindungsvorrichtung (1) nach Anspruch 4, **dadurch gekennzeichnet, dass** der Verschlussabschnitt (14) derart ausgebildet ist, dass dieser bei einer Verlagerung des Dichtkörpers (4) zwischen der Dicht- und der Stauchposition entlang des Fluidkanals (7) in dem Fluidkanal (7) beweglich ist.

6. Medizinische Fluidverbindungsvorrichtung (1) nach einem der vorhergehenden Ansprüche, **dadurch gekennzeichnet, dass** der Dichtkörper (4) einen Profilabschnitt (17a, 17b) aufweist, der derart formschlüssig mit einem komplementären Gegenprofilabschnitt (18a, 18b) des Gehäusekörpers (3) verbunden ist, dass der Dichtkörper (4) in Umfangsrichtung (U) an dem Gehäusekörper (3), insbesondere an der Wandung (8) des Fluidkanals (7), festgelegt ist.

7. Medizinische Fluidverbindungsvorrichtung (1) nach Anspruch 6, **dadurch gekennzeichnet, dass** der Profilabschnitt (17a, 17b) wenigstens ein Gleitelement aufweist, das in einen Führungsschlitz des Gegenprofilabschnitts (18a, 18b) eingreift.

8. Medizinische Fluidverbindungsvorrichtung (1) nach einem der vorhergehenden Ansprüche, **dadurch gekennzeichnet, dass** der Fluidkanal (7) einen Aufnahmeabschnitt (20) aufweist, der zur Aufnahme eines längserstreckten Aufnahmefortsatzes (21) der komplementären Anschlusseinheit (2) vorgesehen ist.

9. Medizinische Fluidverbindungsvorrichtung (1) nach einem der vorhergehenden Ansprüche, **dadurch gekennzeichnet, dass** der Gehäusekörper (3) einen Gewindeabschnitt (22), insbesondere in Gestalt eines Innengewindes, aufweist, der zur Verbindung mit einem Gegengewindeabschnitt (23), insbesondere in Gestalt eines Außengewindes, der komplementären Anschlusseinheit (2) vorgesehen ist.

10. Medizinische Fluidverbindungsvorrichtung (1) nach dem Oberbegriff von Anspruch 1 oder nach einem der vorhergehenden Ansprüche, **dadurch gekennzeichnet, dass** diese aus dem Dichtkörper (4) und dem Gehäusekörper (3) besteht.

## Claims

1. Medical fluid connection device (1) for connecting in a fluid-conducting manner to a complementary connector unit (2), having a housing member (3) having an inlet opening (5) and at least one outlet opening (6, 6a), said inlet opening (5) and said at least one outlet opening (6, 6a) being interconnected in a fluid-conducting manner by way of a fluid duct (7) that extends substantially along an axial direction (Y), and a soft elastic sealing member (4) which in a circumferential direction (U) at least in portions encompasses a wall (8) of the fluid duct (7) and along the axial direction (Y) is guided so as to be movable in relation to the wall (8) of the fluid duct (7), wherein the sealing member (4) in a non-connected state of the fluid connection device (1) assumes a sealing position and seals in a fluid-tight manner the at least one outlet opening (6, 6a) which extends in a substantially radial manner through the wall (8) of the fluid duct (7) and, in a state of the fluid connection device (1) connected to the complementary connector unit (2), in the region of a compression portion (4a), along the axial direction (Y), is elastically compressed to a compression position in such a manner that the at least one outlet opening (6, 6a) is released, wherein a Luer external taper (9) is provided for connecting in a fluid-tight manner to a Luer internal taper (10) of the complementary connector unit (2), **characterized in that** the Luer external taper (9) is configured by a wall portion (11) of the soft elastic sealing member (4).

2. Medical fluid connection device (1) according to claim 1, **characterized in that** the sealing member (4) has at least one passage opening (12a, 12b) which in the compression position in relation to the at least one outlet opening (6, 6a) is disposed so as to at least in portions overlap in such a manner that said at least one outlet opening (6, 6a) is released at least in portions, causing a fluid-conducting connection between the inlet opening (5) and the passage opening (12a, 12b).

3. Medical fluid connection device (1) according to claim 2, **characterized in that** the at least one passage opening (12a, 12b) is configured in the design of a passage slot, and/or the at least one outlet opening (6, 6a) is configured in the design of an outlet slot.

4. Medical fluid connection device (1) according to one of the preceding claims, **characterized in that** the sealing member (4) has a closure portion (14) which in a fluid-tight manner seals an end-side axial opening (15) of the fluid duct (7).

5. Medical fluid connection device (1) according to claim 4, **characterized in that** the closure portion (14) is configured in such a manner that said closure portion (14) in a repositioning of the sealing member (4) between the sealing position and the compression position is movable in the fluid duct (7) along the fluid duct (7).

6. Medical fluid connection device (1) according to one of the preceding claims, **characterized in that** the sealing member (4) has a profiled portion (17a, 17b) which is connected in a form-fitting manner to a complementary profiled counter portion (18a, 18b) of the housing member (3) in such a manner that the sealing member (4) in the circumferential direction (U) is fixed to the housing member (3), in particular to the wall (8) of the fluid duct (7).

7. Medical fluid connection device (1) according to claim 6, **characterized in that** the profiled portion (17a, 17b) has at least one sliding element which engages in a guide slot of the profiled counter portion (18a, 18b).

8. Medical fluid connection device (1) according to one of the preceding claims, **characterized in that** the fluid duct (7) has a receptacle portion (20) which is provided for receiving an elongate receptacle appendage (21) of the complementary connector unit (2).

9. Medical fluid connection device (1) according to one of the preceding claims, **characterized in that** the housing member (3) has a threaded portion (22), in particular in the design of an internal thread, which is provided for connecting to a threaded counter portion (23), in particular in the design of an external thread, of the complementary connector unit (2).

10. Medical fluid connection device (1) according to the preamble of claim 1 or according to one of the preceding claims, **characterized in that** said medical fluid connection device is composed of the sealing member (4) and the housing member (3).

## Revendications

1. Dispositif médical de liaison fluidique (1) pour la liaison fluidique avec une unité de raccordement complémentaire (2), comprenant un corps de boîtier (3) avec une ouverture d'entrée (5) et au moins une ouverture de sortie (6, 6a) qui sont reliées l'une à l'autre fluidiquement par le biais d'un canal fluidique (7) s'étendant essentiellement le long d'une direction axiale (Y), et un corps d'étanchéité élastique souple (4) qui vient en prise au moins en partie autour d'une paroi (8) du canal fluidique (7) dans une direction périphérique (U), et qui est guidé de manière déplaçable le long de la direction axiale (Y) par rapport à la paroi (8) du canal fluidique (7), le corps d'étanchéité (4), dans un état non connecté du dispositif de liaison fluidique (1), adoptant une position d'étanchéité et étanchéifiant de manière étanche aux fluides l'au moins une ouverture de sortie (6, 6a), qui s'étend essentiellement radialement à travers la paroi (8) du canal fluidique (7), et, dans un état du dispositif de liaison fluidique (1) connecté à l'unité de raccordement complémentaire (2), étant écrasé élastiquement dans une position d'écrasement dans la région d'une portion d'écrasement (4a) le long de la direction axiale (Y) de telle sorte que l'au moins une ouverture de sortie (6, 6a) soit libérée, un cône de Luer extérieur (9) étant prévu pour la liaison étanche aux fluides avec un cône de Luer intérieur (10) de l'unité de raccordement complémentaire (2), **caractérisé en ce que** le cône de Luer extérieur (9) est réalisé par une portion de paroi (11) du corps d'étanchéité élastique souple (4).

2. Dispositif médical de liaison fluidique (1) selon la revendication 1, **caractérisé en ce que** le corps d'étanchéité (4) présente au moins une ouverture de passage (12a, 12b) qui, dans la position d'écrasement, est disposée au moins en partie de manière à recouvrir l'au moins une ouverture de sortie (6, 6a) de telle sorte que celle-ci soit au moins en partie libérée et que l'on obtienne une liaison fluidique entre l'ouverture d'entrée (5) et l'ouverture de passage (12a, 12b).

3. Dispositif médical de liaison fluidique (1) selon la revendication 2, **caractérisé en ce que** l'au moins une ouverture de passage (12a, 12b) est réalisée sous forme de fente de passage et/ou l'au moins une ouverture de sortie (6, 6a) est réalisée sous forme de fente de sortie.

4. Dispositif médical de liaison fluidique (1) selon l'une quelconque des revendications précédentes, **caractérisé en ce que** le corps d'étanchéité (4) présente une portion de fermeture (14) qui étanchéifie de manière étanche aux fluides une ouverture axiale (15) côté d'extrémité frontal du canal fluidique (7).

5. Dispositif médical de liaison fluidique (1) selon la revendication 4, **caractérisé en ce que** la portion de fermeture (14) est réalisée de telle sorte que celle-ci, dans le cas d'un déplacement du corps d'étanchéité (4) entre la position d'étanchéité et la position d'écrasement, puisse être déplacée le long du canal fluidique (7) dans le canal fluidique (7).

6. Dispositif médical de liaison fluidique (1) selon l'une quelconque des revendications précédentes, **caractérisé en ce que** le corps d'étanchéité (4) présente une portion profilée (17a, 17b) qui est connectée par engagement par correspondance de formes à une portion profilée conjuguée complémentaire (18a, 18b) du corps de boîtier (3) de telle sorte que le corps d'étanchéité (4), dans la direction périphérique (U), soit fixé au corps de boîtier (3), en particulier à la paroi (8) du canal fluidique (7).

7. Dispositif médical de liaison fluidique (1) selon la revendication 6, **caractérisé en ce que** la portion profilée (17a, 17b) présente au moins un élément de glissement qui s'engage dans une fente de guidage de la portion profilée conjuguée (18a, 18b).

8. Dispositif médical de liaison fluidique (1) selon l'une quelconque des revendications précédentes, **caractérisé en ce que** le canal fluidique (7) présente une portion de réception (20) qui est prévue pour recevoir une saillie de réception allongée (21) de l'unité de raccordement complémentaire (2).

9. Dispositif médical de liaison fluidique (1) selon l'une quelconque des revendications précédentes, **caractérisé en ce que** le corps de boîtier (3) présente une portion filetée (22), en particulier sous la forme d'un filetage intérieur, qui est prévue pour la liaison à une portion filetée conjuguée (23), en particulier sous la forme d'un filetage extérieur de l'unité de raccordement complémentaire (2).

10. Dispositif médical de liaison fluidique (1) selon le préambule de la revendication 1 ou selon l'une quelconque des autres revendications, **caractérisé en ce que** celui-ci se compose du corps d'étanchéité (4) et du corps de boîtier (3).
